# EUROPEAN PATENT SPECIFICATION

(11) **EP 3 043 318 B1**
(45) Date of publication and mention of the grant of the patent: **13.03.2019**
(21) Application number: 16150699.3
(22) Date of filing: 08.01.2016
(51) Int. Cl.: G06T 7/00

(54) **ANALYSIS OF MEDICAL IMAGES AND CREATION OF A REPORT**
ANALYSE MEDIZINISCHER BILDER UND ERZEUGUNG EINES BERICHTS
ANALYSE D'IMAGES MÉDICALES ET CRÉATION D'UN RAPPORT

(30) Priority: 08.01.2015 US 201562101167 P
(43) Date of publication of application: 13.07.2016
(73) Proprietor: Imbio, Minneapolis, MN 55405 (US)
(72) Inventor: MAIER, Cynthia F., Delafield, Wisconsin 53018 (US); AKGUN, Can E., Minneapolis, Minnesota 55403 (US); IVES, Philip S., Deephaven, Minnesota 55391 (US); KOJASOY, Shannon, Minneapolis, Minnesota 55409 (US)
(74) Representative: Elsworth, Dominic Stephen

(56) References cited:
- CA-A1- 2 711 986
- US-A1- 2013 004 043
- LAUGE S PRG ARENSEN ET AL: "Learning COPD Sensitive Filters in Pulmonary CT", 20 September 2009 (2009-09-20), MEDICAL IMAGE COMPUTING AND COMPUTER-ASSISTED INTERVENTION Â ? MICCAI 2009, SPRINGER BERLIN HEIDELBERG, BERLIN, HEIDELBERG, PAGE(S) 699 - 706, XP019130517, ISBN: 978-3-642-04270-6 * abstract * * sections "2 Selection of Training Samples", "5.3 Evaluation", "5.4 Results" *

## Description

### Field of the Invention

**1.** The present disclosure relates to novel and advantageous systems and methods for analyzing medical images of tissue regions and, more particularly, to systems and methods for reporting information regarding the tissue regions to a patient, doctor, or other user.

### Background of the Invention

**2.** Medical imaging has been widely used to assess and monitor the status of various tissue regions within the body. For example, computed tomography (CT) and magnetic resonance imaging (MRI) are 3-dimensional, minimally invasive medical imaging techniques that are capable of providing high contrast images of tissue regions inside the body with excellent spatial resolution. Although these imaging techniques are primarily used qualitatively in the assessment of tissue regions, (i.e., radiologists and other medical professionals visually assess the images and report their findings using qualitative descriptors), recent research has been devoted to the application of computer-aided analysis of CT and MRI images, with the hope that deriving quantitative metrics based on objective criteria can improve diagnosis and/or dictate a more effective treatment strategy for a patient.

**3.** Numerous multi-center clinical trials are currently being conducted, many with tens of thousands of enrolled subjects, to assess whether computer-implemented algorithms may be leveraged to identify characteristic patterns and/or abnormalities on medical images that are correlated with certain diseases or tissue conditions, or are predictive of associated future medical events. Such image characteristics are called "imaging biomarkers." During the course of these studies, medical images from study participants (i.e. study "subjects") are acquired, sometimes serially at multiple time points, and clinical histories and clinical outcomes data for the corresponding subjects are collected. These trials often also collect genetic data, blood samples, and other types of clinical data to test whether additional "biomarkers" such as a specific genetic typing, or a specific blood protein, can be identified to provide more information about a person's current health status or future health outcomes.

**4.** Identifying biomarkers such as imaging characteristics, blood proteins, genetic markers, etc. is expected to allow for more personalized diagnoses and treatment plans. An example of how such a biomarker is used in clinical practice already today is genetic testing for BRCA1 and BRCA2 in women with a family history of breast cancer. The presence of altered forms of these genes results in an increased risk of breast and ovarian cancer. The knowledge that a specific individual has inherited an altered form of these genes is used to drive decisions about whether that individual may be better served by enhanced screening for cancer, prophylactic (risk-reducing) surgery to remove breast tissue, and/or chemo-preventive measures. These decisions are often made in the context of a medical imaging result, wherein the combination of the genetic biomarker information with the presence or non-presence of important imaging biomarkers for cancer (the presence on MR images of a focal area of contrast enhancement with speculated edges, for example) drives clinical decision-making in a more or less aggressive direction depending on the individual patient's likelihood of having cancer.

**5.** CT imaging is used routinely in the chest to assess the pulmonary and surrounding tissues, and is currently the clinical standard of care for identifying anatomical abnormalities in the lungs. In the United States, CT imaging is being adopted for lung cancer screening in high-risk smokers, and European countries are conducting multiple clinical trials to assess the benefits of screening in their populations as well. In the United States alone, it is expected that approximately 7 million high-risk smokers will undergo lung screening using CT every year. Many of these screening participants will have pulmonary nodules that may or may not be cancerous, and most of these participants will suffer from other smoking-related conditions like Chronic Obstructive Pulmonary Disease (COPD) and coronary artery disease. Extracting as much clinical information as possible from these CT images through imaging biomarker analysis would be highly desirable for lung screening participants. By tailoring each patient's care to their individual health status and risks, the clinical and economic benefit of such biomarkers would be very significant as the healthcare burden on society of managing COPD, lung cancer, heart disease, and other smoking-related illnesses is staggering.

**6.** US2013/004043 describes a method, system and product for analysing a sample tissue region of a body to determine the state of the tissue. CA2711986 describes a method and system for measuring tissue damage and disease risk.

The paper by L. Sørensen et al., "Learning COPD Sensitive Filters in Pulmonary CT", Medical Image Computing and Computer-Assisted Intervention MICCAI 2009, pp. 699 - 706, Springer, Berlin, Heidelberg, 20 September 2009, discusses COPD detection from lung CT images using supervised learning. The approach comprises lung segmentation after grouping of CT images according to pulmonary function test results. Reports are produced on the basis of kNN classification.

### Brief Summary of the Invention

The invention is defined in independent claim 1. Advantageous embodiments are defined in the dependent claims.

**7.** The present disclosure, in one or more embodiments, relates to a computer implemented method for assessing and communicating a patient's health status and risk. The method may include the steps of: receiving an imaging dataset of the patient, the imaging dataset comprising a plurality of voxels; automatically analyzing the imaging dataset for the presence and extent of an imaging biomarker; comparing the presence and extent of the imaging biomarker in the imaging dataset of the patient to the presence and extent of the imaging biomarker in historical imaging datasets previously acquired from other patients having known clinical outcomes; using the comparison to calculate personalized quantitative health status and risk metrics for the patient; and creating a report tailored for the intended user of the report to communicate the patient's personalized quantitative health status and risk metrics. In some embodiments the method may also include segmenting the imaging dataset of the patient into voxels corresponding to tissue of interest and voxels corresponding to tissue of no interest. In some embodiments, the imaging biomarker may be the number of voxels with intensity below a threshold. The threshold may be in the range -910 HU to -960 HU. In some embodiments, the quantitative health status and risk metrics may be related to one or any combination of: myocardial infarction, Chronic Obstructive Pulmonary Disease (COPD), emphysema, lung cancer, decreased lung function, COPD exacerbations, coronary artery disease, and stroke.

**8.** Additionally, the present disclosure, in one or more embodiments, relates to a computer implemented method for assessing and communicating a patient's health status and risk. The method may include the steps of: receiving at least one medical image of a patient, the at least one medical image comprising a plurality of voxels; directly comparing the at least one medical image to historical image data previously acquired from other patients having known clinical outcomes; using the comparison to calculate personalized quantitative health status and risk metrics for the patient; and creating a tailored report based on indications of characteristics of the user to communicate the patient's personalized quantitative health status and risk metrics. In some embodiments, the direct comparison may use an unsupervised machine-learning algorithm. In other embodiments, the direct comparison may use a model-based algorithm. The method may further include segmenting the at least one medical image into voxels corresponding to a tissue of interest and voxels not of interest. In some embodiments, the method may further include automatically analyzing the voxels of interest for the presence and extent of an imaging biomarker; and comparing the presence and extent of the imaging biomarker in the voxels of interest to the presence and extent of the imaging biomarker in the historical image data. In some embodiments, the imaging biomarker may be selected from the group of parametric metrics consisting of: number of voxels among the voxels of interest with image intensity below or above a threshold intensity, percentage of voxels of interest with image intensity below or above a threshold intensity, mean image intensity of the voxels of interest, mean image intensity of the voxels among the voxels of interest with image intensity below or above a threshold intensity, standard deviation of the voxels of interest, standard deviation of the image intensity of the voxels among the voxels of interest with image intensity below or above a threshold intensity, other metrics derived from a histogram of the image voxel intensities for the voxels of interest, dimensions of an anatomical feature, pharamacokinetic modeling coefficients of the voxels of interest, and diffusion characteristics of the voxels of interest. Moreover, the group of parametric metrics may include the rate of change of any of the parametric metrics. In some embodiments, the comparison step of the method may include identifying similar imaging features between the patient's at least one medical image and the historical image data. In some embodiments, the similar imaging features may include one or any combination of: textural patterns of image intensity, statistical characteristics of the image intensity distribution, location of focal abnormalities, size of anatomical structure, size of abnormal structure, and physical characteristics of focal abnormalities. In some embodiments of the method, the calculation of personalized quantitative health status and risk metrics may involve clinical data of the patient in addition to the medical image. In some embodiments, image registration techniques may be used to facilitate the comparison of the patient's medical images to the historical image data. Further, the origin of the historical data may be selected from the group consisting of: a multi-center trial, archives of a facility where the patient's medical image is acquired, archives of a facility where the patient is treated, and a purchasable database of medical images and corresponding clinical outcomes. The comparison to historical data may involve other types of clinical data of the patient in addition to the medical image. In some embodiments, the at least one medical image may be from a modality selected from the group consisting of: magnetic resonance imaging, computed tomography, two-dimensional planar x-ray, x-ray mammography, positron emission tomography, ultrasound, and single-photon emission computed tomography.

### Brief Description of the Drawings

**9.** While the specification concludes with claims particularly pointing out and distinctly claiming the subject matter that is regarded as forming the various embodiments of the present disclosure, it is believed that the invention will be better understood from the following description taken in conjunction with the accompanying Figures, in which:

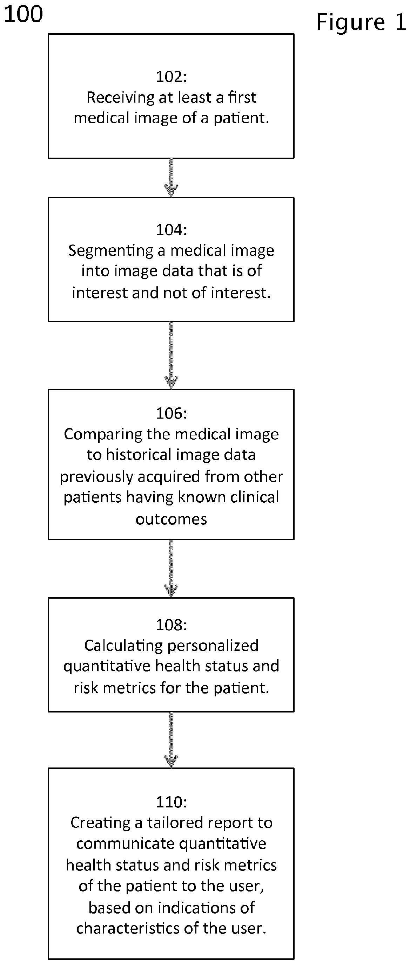

**10.** FIG. 1 is a flow chart of a method of analyzing one or more medical images and creating one or more reports, in accordance with at least one embodiment of the present disclosure.

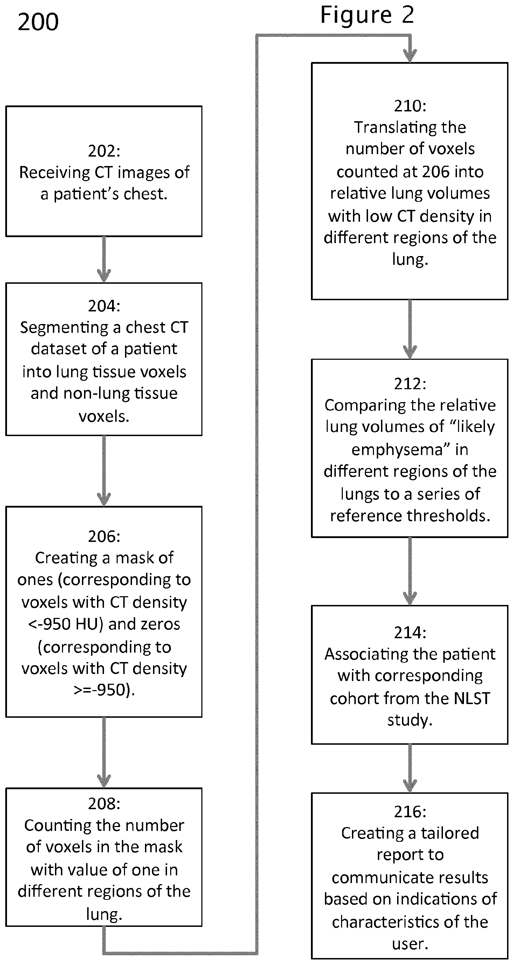

**11.** FIG. 2 is a flow chart of a method of analyzing one or more medical images related to lung screening and creating one or more reports, in accordance with at least one embodiment of the present disclosure.

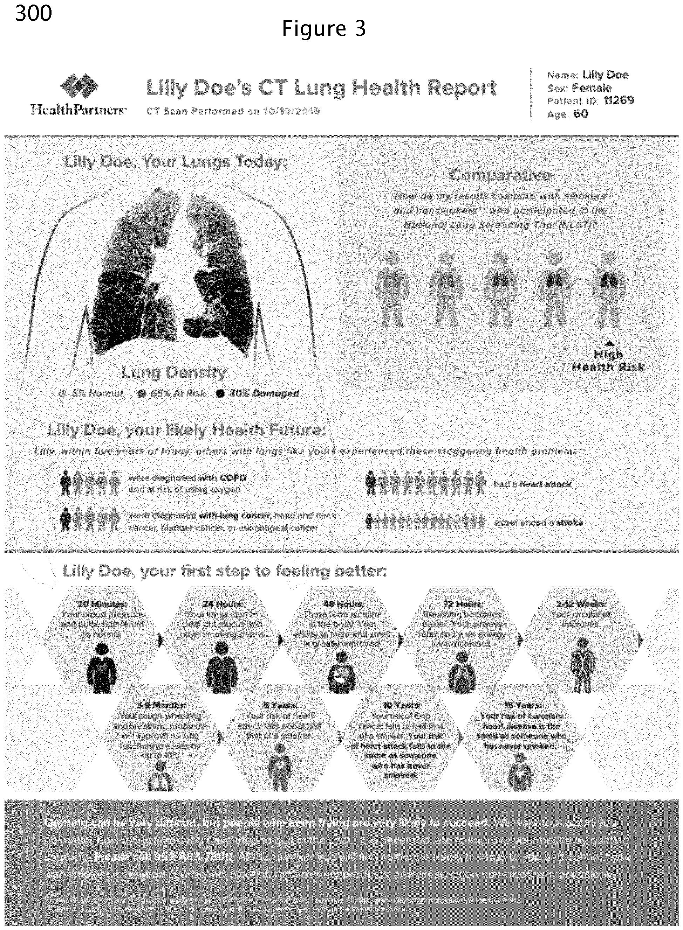

**12.** FIG. 3 is an example of a report for a patient generated by the exemplary method of FIG. 2.

### Detailed Description

**13.** The present disclosure relates to computer-implemented systems and methods for automatically analyzing a patient's one or more medical images and creating at least one report that provides quantitative metrics related to the patient's current health status and their risks for future health outcomes. The analysis may be based on a computer-implemented algorithm that compares the patient's images to one or more comparison images of the same or similar tissue regions from one or more other individuals for whom the corresponding health status and/or outcomes are known. Multiple reports may be created for the same patient from the same medical images, wherein each of the multiple reports may be differently tailored for different types of intended users of the reports. As used herein, a "user" may be, for example, a patient, a patient's guardian or caregiver, a primary care physician, a nurse, a nurse practitioner, a chiropractor or osteopathic practitioner, a radiologist, a radiology technician, a specialist physician in the patient's disease, a surgeon, an interventional radiologist, a nutritionist, a dietician, a physician's assistant, an insurance company, a government body, or any other medical, dental or health professional.

**14.** The medical image(s) or medical image data for the patient's image(s) as well as the comparison image(s) may be from a variety of different sources, including, but not limited to magnetic resonance imaging (MRI), computed tomography (CT), two-dimensional planar x-ray (either plain film converted to digital, or digital x-ray images), X-ray mammography, positron emission tomography (PET), ultrasound (US), or single-photon emission computed tomography (SPECT). Within a given instrumentation source (i.e. MRI, CT, X-Ray, PET, SPECT or other instrumentation source) a variety of data can be generated. In order to compare images of the same patient between different time points or to compare images of different patients, imaging data, irrespective of source and modality, can be quantified (i.e., made to have physical units) or normalized (i.e., scaled so that the pixel intensities fall within a known range based on an external phantom, something of known and constant property, or a defined signal within the image volume).

**15.** The patient's image(s) as well as the comparison image(s) may be anatomical images in nature, or they may be functional images that provide information about the tissue physiology or functioning, for example, functional MRI exams of the brain, dynamic contrast-enhanced MRI exams, PET imaging, high temporal resolution imaging during motion of a joint, diffusion MRI, MRI elastography, contrast-enhanced US, etc. As used herein, "medical image" is also contemplated to include medical imaging data that is acquired as a step to creating (or "reconstructing") a medical image, for example raw imaging data, CT sinograms, MRI k-space data, etc.

**16.** The systems and methods of the present disclosure are not limited to a particular type or kind of tissue region. By way of example only, suitable tissue types include lung, prostate, breast, colon, rectum, bladder, ovaries, skin, liver, spine, bone, pancreas, cervix, lymph, thyroid, spleen, adrenal gland, salivary gland, sebaceous gland, testis, thymus gland, penis, uterus, trachea, skeletal muscle, smooth muscle, heart, brain, bone, etc. In some embodiments, the tissue region may be a whole body or large portion thereof (for example, a body segment such as a torso or limb; a body system such as the gastrointestinal system, endocrine system, etc.; or a whole organ comprising multiple tumors, such as whole liver) of a living human being. In some embodiments, the tissue region may be a diseased tissue region. In some embodiments, the tissue region may be an organ. In some embodiments, the tissue region may be a tumor, for example, a malignant or benign tumor. In some embodiments, the tissue region may be a breast tumor, a liver tumor, a bone lesion, and/or a head/neck tumor. In some embodiments, the tissue may be from a non-human animal.

**17.** In some embodiments, the systems and methods of the present disclosure may be used for screening for disease, prognosis or diagnosis of diseases, base-line assessments, treatment planning, treatment follow-up, or other user education regarding tissue state. In addition, the systems and methods are not limited to a particular disease, pathology, or type of treatment. In some embodiments, the systems and methods may be used as part of a pharmaceutical treatment, a vaccine treatment, a chemotherapy based treatment, a radiation based treatment, a surgical treatment, a homeopathic treatment, other treatment, and/or a combination of treatments.

**18.** The systems and methods of the present disclosure may comprise a parametric response map (PRM) in some embodiments. Methods and systems for creating parametric response maps and obtaining quantitative data therefrom are described in U.S. Pat. Appln. No. 13/539,232, entitled, "Pixel and Voxel-Based Analysis of Registered Medical Images for Assessing Bone Integrity," filed June, 29, 2012; U.S. Pat. No. 8,185,186, entitled, "Systems and Methods for Tissue Imaging," issued May 22, 2012; U.S. Appln. No. "Systems and Methods for Tissue Imaging," filed May 2, 2012; U.S. Pat. No. 13/539,254, entitled, "Tissue Phasic Classification Mapping System and Method," filed June 29, 2012; U.S. Pat. Appln. No. 13/683,746, entitled "Voxel-Based Approach for Disease Detection and Evolution," filed November 21, 2012.

**19.** In some embodiments, the systems and methods of the present disclosure may compare a patient's medical image(s) indirectly to one or more comparison images by analyzing the patient's medical image(s) for the presence and/or extent/amount of an imaging biomarker. Such imaging biomarker be defined based on the comparison image(s) of the same or similar tissue regions from the other individual(s) for whom the corresponding health status and/or outcomes are known. In some embodiments, the imaging biomarker may have been previously defined. The extent to which the biomarker is present or not present in the patient's image(s) may be translated into quantitative metrics related to the patient's current health status and/or their risks for future health outcomes. In some embodiments, other types of clinical data may be included in the calculation of the quantitative status and/or risk metrics, i.e. the algorithm may include both imaging biomarker as well as other types of biomarker data in the calculation of the quantitative status and/or risk metrics. The patient's risk metrics may be calculated by identifying a particular subset of individuals represented by the comparison image(s) who share the same or similar imaging biomarker status as the patient (i.e. a "corresponding cohort") and determining the prevalence of a particular outcome in this cohort. If the prevalence of the particular outcome in the corresponding cohort is higher or lower than a normal population with statistical significance, the risk metrics may be calculated for the particular outcome for the patient based on the corresponding prevalences in the cohort.

**20.** One example of such an imaging biomarker could be the relative volume of low-density tissue in the upper lobes of the lungs on a patient's CT images. From an analysis of comparison images, such as previously obtained CT images of the lungs from other individuals for whom the corresponding health status and/or outcomes are known, it may be determined with high statistical significance that the prevalence of lung cancer is five times (5x) higher in patients who have more than 10% relative volume of low density tissue in the upper lobes of their lungs compared to patients with no low density tissue (i.e. normal patients). This fact could be translated into a risk factor of "5x greater than normal" for lung cancer for patients with greater than 10% relative volume of low-density tissue in the upper lobes of the lung. For this example, the presence of greater than 10% relative volume of low density tissue in the upper lobes of the lung is an imaging biomarker for lung cancer, with "5x greater than normal" as the quantitative risk metric. In this example, a computer-implemented algorithm of the present disclosure may measure, automatically and without any user input or intervention, the relative volume of low-density tissue in the upper lobes of the lung, and calculate a risk metric of "5x greater than normal" for those patients with results greater than 10%. From other previous research studies, it may also have been determined that low density tissue in the upper lobes of the lung is associated with emphysema on histopathology 90% of the time, i.e. the regions of low density tissue may be considered as "likely emphysema." The relative volume of "likely emphysema" in that patient's lungs may additionally be an example of a quantitative metric of current health status in this example.

**21.** In other embodiments, the systems and methods of the present disclosure may perform an on-the-fly comparison of a patient's image(s) to comparison image(s) of the same or similar tissue regions from one or more other individuals for whom the corresponding health status and/or outcomes are known. The on-the-fly comparison may comprise calculating metrics related to the degree of similarity between the patient's medical image(s) and the comparison image(s) (a similarity matrix, for example), and identifying a corresponding cohort of the one or more individuals represented by the comparison image(s) whose image(s) are the most similar to the patient's image(s) based on these similarity metrics. Additionally or alternatively, the on-the-fly comparison may comprise identifying similar imaging features between the patient's medical image(s) and the comparison image(s), such as for example, similar textural patterns of image intensity, similar distribution characteristics of CT densities in a tissue region (e.g. mean CT density of lung tissue, or CT density histogram skew or principal component analysis metrics, etc.), focal abnormalities in similar locations, size of an anatomical feature (e.g. hippocampal volume on MR images of the brain), similar characteristics of focal abnormalities (e.g. lobulated or speculated edges of a contrast-enhancing region on MR images of the breast, partial solidity of a pulmonary nodule detected on CT images of the lung, etc.) and identifying a corresponding cohort. The quantitative status and/or risk metrics for the patient may then be calculated based on the known health status and/or outcomes for the corresponding cohort, as described in the previous example.

**22.** The on-the-fly image comparison may be constrained by using a priori medical information (e.g., model-based algorithms) or may be unconstrained (e.g., neural networks and other unsupervised machine-learning algorithms). The comparison may include all the voxel data in all the images in the analysis or it may extract only particular regions or anatomies of interest from the image(s). The comparison may be based on the reconstructed image data, or earlier forms of the imaging data, such as for example, CT sinograms, or MRI k-space data. The on-the-fly comparison may comprise solely a comparison of the patient's image(s) to the comparison image(s), or it may additionally comprise a comparison of additional other types of clinical data, such as for example genetic data, blood proteins, etc.

**23.** An algorithm of the present disclosure may comprise a sophisticated image registration technique to facilitate comparison between a patient's one or more images and one or more comparison images from one or more other individuals. It may additionally comprise an indirect comparison between the patient's image(s) and the comparison image(s), via comparison to an "anatomical atlas" generated from averaging or otherwise combining anatomical data from the comparison image(s). The algorithm may additionally or alternatively comprise comparing the patient's image(s) to the patient's own image(s), such as previously obtained image(s) for the patient, and calculating values related to the change in the patient's own image(s) (rate of change in size of multiple sclerosis plaques visualized on MR images, for example), before comparing these values to the comparison image(s) from the one or more other individuals.

**24.** In an example of one embodiment of the invention, the patient is a high-risk smoker, the patient's medical images are CT lung screening images acquired at the patient's annual screening visit, and the comparison images were collected from other individuals during a large, multi-center clinical trial, for example, the National Lung Screening Trial (NLST) or the COPDGene Trial. A computer-implemented analysis of the comparison images was completed on an occasion prior to the patient's annual screening visit, at which prior occasion biomarkers related to the distribution of CT densities in the lung parenchyma were defined and correlated quantitatively to important clinical outcomes such as the presence and extent of emphysema, risk for future decline in lung function, risk for number of future acute exacerbations requiring hospitalization, risk for current and future lung cancer, risk for current and future coronary artery disease, risk for future stroke, etc. Multiple reports providing these quantitative metrics related to the presence and extent of emphysema (i.e., patient's current health status) and the patient's risks for the future health outcomes are generated for different users, each of which reports is tailored for its specific intended user.

**25.** Continuing with the above example, a report for the patient's referring physician may include a CT image with a transparent color overlay indicating areas of "likely emphysema" (defined in the algorithm for example, as areas with >90% risk of being emphysema) as well as detailed quantitative measures of the amount of likely emphysema present (e.g., statistics for the amount of likely emphysema present in each individual lung and individual lobar segments of each lung), as well as quality metrics related to the measurement accuracy of the quantitative metrics and references to normal ranges. The report may also include a complete new set of images generated by the computer-implemented algorithm with transparent color overlays on the original gray-scale CT images to indicate the areas of likely emphysema. These images may be used by the referring physician to plan an interventional procedure, such as for example implantation of a valve or pulmonary coil or a biopsy procedure by facilitating the physician's quick assessment of the distribution of likely emphysema in the planned area of the procedure. In contrast, a second report for the patient his/herself may include only a simple image of the patient's lungs, comprising only the portion of their CT images that correspond to lung tissue (i.e. with all background removed as shown in FIG. 3, for example), wherein the lung image is re-colored to fit a lay person's conception of lung health with the normal portion of their lungs shown as pink to indicate normal tissue, and the likely emphysema portion of the lungs shown as black to indicate a diseased state, and an outline of a torso is shown surrounding the lung in order to provide a reference for the patient to their own anatomy. Very simple personalized quantitative health status and risk metrics may be included, using lay language such as "Mary Doe, if you continue smoking, your risk for developing lung cancer in the next 5 years is greater than 50%", or "Mary Doe, 1 in 3 people with lungs similar to yours developed lung cancer in 5 years if they continued smoking." The report for the patient may contain only very simple lay language and minimal quantitative metrics. It may also be designed specifically to have maximum impact on the patient's perception of their personal risk from continuing to smoke cigarettes, and to motivate them to make a quit attempt by including messaging like for example, "Quitting smoking is difficult, but we are here to help you," and including a phone number for a Smoking Cessation Counseling Center.

**26.** FIG. 1 shows a method for analyzing one or more medical images and creating a report, according to some embodiments. At least as shown, the computer-implemented method 100 comprises the computer-automated steps of: receiving at least a first medical image of a patient, said medical image having been obtained from a medical imaging system, as shown at 102; optionally segmenting the image into image data that is of interest and image data that is not of interest, as shown at 104; analyzing the image data of interest by comparing it to comparison image data, as shown at 106; based on the comparison performed at step 106, calculating quantitative metrics related to the patient's current health status and/or their risks for future health outcomes, as shown at 108; and automatically by computer software generating a report providing the results of the analysis of the image data of interest, including the quantitative health status and/or risk metrics calculated at step 108, wherein the reporting format is specifically tailored to the intended user, as shown at 110.

**27.** Segmenting the image into image data that is of interest and image data that is not of interest at step 104 may include distinguishing image data that is relevant for a particular analysis, diagnosis, prognosis, assessment, treatment planning, treatment follow-up, or other determination. For example, in some embodiments, separating image data may include segmenting lung paraenchyma from the background anatomy and discarding the background anatomy for the purpose of calculating the quantitative status and risk metrics. In some embodiments, the image data of interest may be the entire image and in other embodiments, the image data of interest may be a subset of the image data.

**28.** The comparison image data used for comparison in step 106 may be from one or more images of the same or similar tissue regions from other individual(s) for whom the corresponding health status and/or outcomes are known. The comparison images may be obtained in various manners. For example, in some embodiments, the one or more individuals represented by the comparison images may be study participants from a multicenter clinical trial. In other embodiments, the one or more individuals may be patients who were imaged previously at the same facility as the patient and whose health status at the time of imaging as well as subsequent health outcomes are known. In other embodiments, the one or more individuals may be patients from elsewhere whose collective image(s) have been bundled with their corresponding health status and/or outcomes into a database, such as a purchasable database, or provided for use by a vendor, or are customers of a health insurance company for example. In other embodiments, the comparison image(s) may be obtained from other sources. Further, it may be appreciated that the comparison image(s) may be obtained at any point in time with respect to the systems and methods of the present disclosure. For example, the comparison image(s) may be obtained or received prior to step 102 wherein the patient's image(s) are received. In other embodiments, the comparison image(s) may be obtained after the patient's image(s) are received or obtained. In still further embodiments, the comparison image(s) may be obtained substantially simultaneously with the patient's image(s).

**29.** In some embodiments, step 106 of analyzing the image data of interest by comparing it to comparison image data may comprise identifying a corresponding cohort of the one or more individuals represented by the comparison image(s) whose imaging data is most similar to the current patient's imaging data. Similarly, step 108 of calculating quantitative metrics related to the patient's current health status and/or their risks for future health outcomes may comprise using the known health status and/or prevalence of outcomes in the corresponding cohort of the one or more individuals represented by the comparison images to calculate the patient's quantitative metrics of health status and health risks.

**30.** In some embodiments, step 106 may comprise analyzing the image data of interest for the presence and/or extent of known imaging biomarkers as an indirect form of comparison to comparison image(s). By way of example, but not limitation, these imaging biomarkers may be: characteristic patterns of intensity values (e.g., percentage or volume of voxels below or above a threshold value, mean image intensity below or above a threshold value, skew of a histogram of image voxel intensities, value for a certain percentile of the image voxel intensity histogram, etc.), image textures (e.g., reticular pattern, honeycomb pattern, ground glass opacities, etc. on CT lung images), dimensions or amount of an anatomical feature (e.g., relative dimensions of the right versus left ventricle of the heart on CT images, relative volume of gray to white matter in the brain on MR images, arterial wall thickness, size or relative fraction of ductal tissue in the breast on mammography images, carotid artery diameter, size of a coronary artery calcification, etc.), other measurable characteristics of anatomical features (e.g., number of branches in the bronchial tree detectable on CT images, quantitative metrics derived from a fractal analysis of diffusion tensor imaging in the brain, location of a thrombus or embolism, etc.), or physiological metrics derived from functional imaging exams (e.g., perfusion/diffusion mismatch in MR brain imaging after a suspected acute stroke, metrics related to contrast agent uptake in MR or CT in tumors, or other vascularized tissues, etc.).

**31.** In other embodiments, step 106 may comprise a direct on-the-fly comparison of the image data of interest to comparison image data. This comparison may comprise any type of algorithm that tests for similarities between the image data of interest and the comparison image data, including unsupervised machine learning algorithms of all varieties. Step 106 may result in the identification of a cohort of the one or more individuals represented by the comparison image(s) whose image data is most similar to the patient's image data. It may not be necessary that a specific "closed" cohort of individuals be identified at this step. Rather, step 106 may comprise "indexing" the patient into the population of one or more individuals represented by the comparison image(s), wherein the individuals are distributed on a continuous spectrum of relative similarity to the patient on the basis of their image data.

**32.** In some versions, step 106 comprises an indirect form of comparison to comparison image(s), via a comparison to an anatomical reference atlas (or physiological reference atlas) that has been constructed as an average or other composite of the comparison image(s). For example, it may be desirable to compare a patient's hippocampal volume to normal age-matched reference subjects by automatically registering the patient's brain MR images to an anatomical atlas of MR images and segmenting the hippocampus for volumetric measurement.

**33.** In some embodiments, step 106 may additionally or alternatively comprise comparing other clinical data for the patient to comparison clinical data. For example, clinical data for the patient may be compared to clinical data for the one or more individuals represented by the comparison images. Such clinical data may include, for example, blood protein or metabolite measures, genetic data, etc. Other patient-specific characteristics may also be included in this comparison, for example, patient age, patient sex, patient ethnicity, patient weight, patient height, patient body mass index, patient smoking history, history of prior disease, patient family history of disease, or other such patient-specific information.

**34.** In some embodiments, step 108 may comprise calculating one or more health status metrics that are simply measured anatomical or physiological quantities. One example of a simple health status metric is the relative change in internal diameter of the carotid artery along its length in an area of stenosis, as this may provide an indirect measurement of the burden of plaque in the carotid artery wall. For patients with a stenosis greater than a threshold percentage of the normal arterial diameter, the risk of having a stroke as a possible future outcome may be elevated compared to normal people, and a carotid endarterectomy may be recommended to remove the plaque material and prevent the stroke from occurring. Note that the measurement of the internal diameters of the carotid artery may be accomplished after the comparison of the patient's image data, either indirectly or directly at step 106, to comparison image(s), and the threshold value and corresponding risk metric for stroke may be determined if the corresponding health status and outcomes are known for the one or more individuals represented by the comparison image(s). In other embodiments, the health status metrics may be more complex calculations comparing a patient's data to a reference population, or identifying actual presence of burden of disease. In other embodiments, the health status metrics may be calculated by comparing a patient's image data to their own image data that was acquired on a prior occasion, for example, it is often desirable to monitor the size of a patient's brain ventricles to determine whether excess cerebrospinal fluid has collected in the ventricles and a brain shunt should be placed. In this example, the patient's relevant health status metric may be any changes in size to their own brain ventricles during the period of monitoring.

**35.** In some embodiments, a software application may be used to create the report of step 110. For example, in some embodiments, a user-readable report may be created at step 110 within the same software application used to perform any of the preceding steps. In other embodiments, a first software application may report the quantitative health status and/or risk metrics in a predetermined format for automated processing by a second software application that creates the user-readable report. In at least one embodiment, the method may write the quantitative health status and/or risk metrics to a file according to a data file format that has been previously defined. This data file format may be previously defined by the input requirements of a voice recognition software application, which converts the speech of a radiologist to text and then combines the text with the contents of the data file to create a user-readable report for review by a user.

**36.** The report format may include a printed report, an electronic report, a saved report, an emailed report, tablet device, smartphone, or wearable tech with a display interface such as an optical head-mounted display. The report may be uploaded by the method to a cloud storage bank for retrieval by a user. Additionally or alternatively, the report may be stored or wrapped in a second file format for communication to and display on a user device. For example, the reporting may take the form of generating a PDF report which is wrapped in a DICOM or HL7 wrapper for communication to a Picture Archiving and Communication System (PACS) where it can be accessed and read by a user.

**37.** FIG. 2 shows an example implementation of the method of the present disclosure. Specifically, the method 200 of FIG. 2 may provide for image analysis and report creation for a patient's lung imaging, such as for an annual lung screening CT imaging exam. At least as shown, the computer-implemented method 200 may comprise the computer-automated steps of: receiving one or more CT images of a patient's chest, said CT images having been obtained from a CT scanner, as shown at 202; segmenting the images into voxels corresponding to lung parenchyma from other types of tissue, as shown at 204; creating a mask of ones and zeroes corresponding to lung parenchyma voxels with CT density less than a threshold value of -950 Hounsfield Units, said threshold having been previously defined as a biomarker for "likely emphysema," as shown at 206; counting the number of lung parenchyma voxels with mask values = 1 in each lung, and in the different lobes of each lung, as shown at 208; translating the values calculated at 208 into relative lung volumes of "likely emphysema," as shown at 210; comparing the relative volumes of "likely emphysema" to a series of CT density thresholds, as shown at 212; calculating the patient's risk for the relevant clinical outcomes by associating the patient with one of the multiple participant cohorts according to the patient's relative volume of "likely emphysema," as shown at 214; and creating by computer software a patient-centered report, wherein the reporting format is specifically tailored for the average lung screening patient, such as for example a lay person with a grade 6 reading level, as shown at 216.

**38.** In some embodiments, the CT density thresholds of step 212 may include one or more density thresholds defined on a prior occasion from an analysis of one or more images, such as images from the NLST image database or another image database. The density threshold(s) may be defined by stratifying the one or more images into individual relative volumes of "likely emphysema," and defined multiple thresholds to create multiple separate corresponding cohorts having similar prevalence of relevant clinical outcomes, e.g. lung cancer.

**39.** In some embodiments, steps 204 to 214 may additionally comprise evaluating the patient's image(s) for other biomarkers. For example, the presence of coronary artery calcifications, and the quantitative health status and risk metrics may reflect these additional biomarkers as well. In other words, the identification of an appropriate corresponding cohort may comprise considering additional imaging or other biomarker or patient-characteristic information, for example, patient age, smoking history, etc.

**40.** FIG. 3 is an example of a report for a patient generated by the exemplary method of FIG. 2 and is included for illustrative purposes only. As shown, the contents and format of the report may be tailored to encourage the patient to make an attempt to quit smoking, i.e. the patient report may be intended to enhance smoking cessation counseling. The example report of FIG. 3 has five sections of information addressing all components of the Health Belief Model, which is an accepted model for influencing a patient's health behaviors: (1) an Image section, which provides visual feedback and quantitative health status metrics related to the areas of "likely emphysema"; (2) a Comparative section, which contains quantitative health risk metrics derived from a comparison of the patient's images to the study participants in the NLST study; (3) a Health Outcomes section which provides information about the long-term health outcomes for the associated participant cohort; (4) a Quit Now section which outlines the benefits of making a quit attempt; and (5) an Outreach section, which provides contact information for a Smoking Cessation Support service. To ensure that the report may be understood by the average participant in a lung screening program, the level of English used may be targeted to grade 6 proficiency level and the amount of text content may be dramatically reduced compared to a report that would be intended for a healthcare professional. As shown, the patient's individual health risks may be included using graphical means to convey the risks instead of percentages or text. It may be appreciated that in other embodiments, the tailored report may have other sections, graphics, language, goals, and/or other elements.

**41.** In some examples, the method shown in FIG. 2 may a create a patient report whose format and content is additionally tailored to the patient on the basis of one or more indications of characteristics of the patient. A report for a younger patient may differ from a report for an older patient, for example; a report for a woman may differ from a report for a man; and reports may differ based on race, other genetic characteristics, or behavioral characteristics of the patient, which may affect the impact of the report on the patient's perceptions and response based on behavioral research. The report format and content may be specifically tailored on the basis of the individual patient's characteristics to be most effective for influencing that patient's behavior. For example, the font size may be increased for patients of advanced age. Different messaging may be selected to be more effective in younger patients versus older patients. For example, younger patients may be more influenced to quit smoking by a message that emphasizes their increased risk for developing advanced emphysema and the concomitant loss of earning potential, chronic health issues, etc. As another example, male patients may be more influenced by messaging that focuses on their increased risk of heart disease.

**42.** In some implementations, the method may receive the medical images directly as a DICOM send/push from a medical imaging system, or via a secondary routing decision by a separate device based on the contents of the DICOM tags in the medical images, or via a manual push by a user. The method may be implemented as a software application running in a hospital enterprise data center, and may be installed as a virtual machine on a virtualization layer such as VMware. The method may include the capability of processing in parallel to generate reports from multiple patients concurrently. Multiple reports for multiple patients may also be generated concurrently. The method may comprise a step of dynamically activating additional virtual machines to provide the hardware resources necessary to process the incoming patient images in parallel, and then dynamically de-activating these virtual machines when they are not needed.

**43.** In some embodiments, the method may route the report(s) automatically to a predetermined receiving device such as a Picture Archiving and Communications system (PACS) or an Enterprise Health Record (EHR) system for access and review by a user. In other embodiments, the method may save the quantitative health status and risk metrics in a text file format report according to a pre-determined data ordering, for later parsing by a different software application into a user-readable report.

**44.** The computer-implemented methods of this disclosure may utilize any computer-based system in order to compute, calculate, retrieve, reproduce, transform, handle or otherwise utilize any of the retrieved data. For purposes of this disclosure, any system or information handling system used for the methods described herein may include any instrumentality or aggregate of instrumentalities operable to compute, calculate, determine, classify, process, transmit, receive, retrieve, originate, switch, store, display, communicate, manifest, detect, record, reproduce, handle, or utilize any form of information, intelligence, or data for business, scientific, control, or other purposes. For example, a system or any portion thereof may be a personal computer (e.g., desktop or laptop), tablet computer, mobile device (e.g., personal digital assistant (PDA) or smart phone), server (e.g., blade server or rack server), a network storage device, or any other suitable device or combination of devices and may vary in size, shape, performance, functionality, and price. A system may include random access memory (RAM), one or more processing resources such as a central processing unit (CPU) or hardware or software control logic, ROM, and/or other types of nonvolatile memory. Additional components of a system may include one or more disk drives or one or more mass storage devices, one or more network ports for communicating with external devices as well as various input and output (I/O) devices, such as a keyboard, a mouse, touchscreen and/or a video display. Mass storage devices may include, but are not limited to, a hard disk drive, floppy disk drive, CD-ROM drive, smart drive, flash drive, or other types of non-volatile data storage, a plurality of storage devices, or any combination of storage devices. A system may include what is referred to as a user interface, which may generally include a display, mouse or other cursor control device, keyboard, button, touchpad, touch screen, microphone, camera, video recorder, speaker, LED, light, joystick, switch, buzzer, bell, and/or other user input/output device for communicating with one or more users or for entering information into the system. Output devices may include any type of device for presenting information to a user, including but not limited to, a computer monitor, flat-screen display, or other visual display, a printer, and/or speakers or any other device for providing information in audio form, such as a telephone, a plurality of output devices, or any combination of output devices. A system may also include one or more buses operable to transmit communications between the various hardware components.

**45.** One or more programs or applications, such as a web browser, and/or other applications may be stored in one or more of the system data storage devices. Programs or applications may be loaded in part or in whole into a main memory or processor during execution by the processor. One or more processors may execute applications or programs to run systems or methods of the present disclosure, or portions thereof, stored as executable programs or program code in the memory, or received from the Internet or other network. Any commercial or freeware web browser or other application capable of retrieving content from a network and displaying pages or screens may be used. In some embodiments, a customized application may be used to access, display, and update information.

**46.** Hardware and software components of the present disclosure, as discussed herein, may be integral portions of a single computer or server or may be connected parts of a computer network. The hardware and software components may be located within a single location or, in other embodiments, portions of the hardware and software components may be divided among a plurality of locations and connected directly or through a global computer information network, such as the Internet.

**47.** As will be appreciated by one of skill in the art, the various embodiments of the present disclosure may be represented as a method (including, for example, a computer-implemented process, a business process, and/or any other process), apparatus (including, for example, a system, machine, device, computer program product, and/or the like), or a combination of the foregoing. Accordingly, embodiments of the present disclosure may take the form of an entirely hardware embodiment, an entirely software embodiment (including firmware, middleware, microcode, hardware description languages, etc.), or an embodiment combining software and hardware aspects. Furthermore, embodiments of the present disclosure may take the form of a computer program product on a computer-readable medium or computer-readable storage medium, having computer-executable program code embodied in the medium, that define processes or methods described herein. A processor or processors may perform the necessary tasks defined by the computer-executable program code. Computer-executable program code for carrying out operations of embodiments of the present disclosure may be written in an object oriented, scripted or unscripted programming language such as Java, Perl, PHP, Visual Basic, Smalltalk, C++, or the like. However, the computer program code for carrying out operations of embodiments of the present disclosure may also be written in conventional procedural programming languages, such as the C programming language or similar programming languages. A code segment may represent a procedure, a function, a subprogram, a program, a routine, a subroutine, a module, an object, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

**48.** In the context of this document, a computer readable medium may be any medium that can contain, store, communicate, or transport the program for use by or in connection with the systems disclosed herein. The computer-executable program code may be transmitted using any appropriate medium, including but not limited to the Internet, optical fiber cable, radio frequency (RF) signals or other wireless signals, or other mediums. The computer readable medium may be, for example but is not limited to, an electronic, magnetic, optical, electromagnetic, infrared, or semiconductor system, apparatus, or device. More specific examples of suitable computer readable medium include, but are not limited to, an electrical connection having one or more wires or a tangible storage medium such as a portable computer diskette, a hard disk, a random access memory (RAM), a read-only memory (ROM), an erasable programmable read-only memory (EPROM or Flash memory), a compact disc read-only memory (CD-ROM), or other optical or magnetic storage device. Computer-readable media includes, but is not to be confused with, computer-readable storage medium, which is intended to cover all physical, non-transitory, or similar examples of computer-readable media.

**49.** Various embodiments of the present disclosure may be described herein with reference to flowchart illustrations and/or block diagrams of methods, apparatus (systems), and computer program products. It is understood that each block of the flowchart illustrations and/or block diagrams, and/or combinations of blocks in the flowchart illustrations and/or block diagrams, can be implemented by computer-executable program code portions. These computer-executable program code portions may be provided to a processor of a general purpose computer, special purpose computer, or other programmable data processing apparatus to produce a particular machine, such that the code portions, which execute via the processor of the computer or other programmable data processing apparatus, create mechanisms for implementing the functions/acts specified in the flowchart and/or block diagram block or blocks. Alternatively, computer program implemented steps or acts may be combined with operator or human implemented steps or acts in order to carry out an embodiment of the invention.

**50.** Additionally, although a flowchart may illustrate a method as a sequential process, many of the operations in the flowcharts illustrated herein can be performed in parallel or concurrently. In addition, the order of the method steps illustrated in a flowchart may be rearranged for some embodiments. Similarly, a method illustrated in a flow chart could have additional steps not included therein or fewer steps than those shown. A method step may correspond to a method, a function, a procedure, a subroutine, a subprogram, etc.

**51.** As used herein, the terms "substantially" or "generally" refer to the complete or nearly complete extent or degree of an action, characteristic, property, state, structure, item, or result. For example, an object that is "substantially" or "generally" enclosed would mean that the object is either completely enclosed or nearly completely enclosed. The exact allowable degree of deviation from absolute completeness may in some cases depend on the specific context. However, generally speaking, the nearness of completion will be so as to have generally the same overall result as if absolute and total completion were obtained. The use of "substantially" or "generally" is equally applicable when used in a negative connotation to refer to the complete or near complete lack of an action, characteristic, property, state, structure, item, or result. For example, an element, combination, embodiment, or composition that is "substantially free of' or "generally free of' an ingredient or element may still actually contain such item as long as there is generally no measurable effect thereof.

**52.** As used herein any reference to "one embodiment" or "an embodiment" means that a particular element, feature, structure, or characteristic described in connection with the embodiment is included in at least one embodiment or implementation. The appearances of the phrase "in one embodiment" in various places in the specification are not necessarily all referring to the same embodiment. Features, elements, structures, or characteristics described with respect to different embodiments may be combined in any suitable combination.

**53.** As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

**54.** In addition, use of the "a" or "an" are employed to describe elements and components of the embodiments herein. This is done merely for convenience and to give a general sense of the description. This description should be read to include one or at least one and the singular also includes the plural unless it is obvious that it is meant otherwise.

**55.** Still further, the figures depict preferred embodiments for purposes of illustration only. One skilled in the art will readily recognize from the discussion herein that alternative embodiments of the structures and methods illustrated herein may be employed without departing from the principles described herein.

**56.** While the systems and methods described herein have been described in reference to some exemplary embodiments, these embodiments are not limiting and are not necessarily exclusive of each other, and it is contemplated that particular features of various embodiments may be omitted or combined for use with features of other embodiments while remaining within the scope of the claims.

## Claims

1. A computer implemented method for assessing and communicating a patient's health status and risk, the method comprising:
receiving an imaging dataset of a patient (102), the imaging dataset comprising a plurality of voxels;
directly comparing the imaging dataset to historical imaging datasets previously acquired from other patients having known clinical outcomes (106);
using the comparison to calculate personalized quantitative health status and risk metrics for the patient (108); and
creating a tailored report based on indications of characteristics of the user to communicate the patient's personalized quantitative health status and risk metrics (110).

2. The method of claim 1, wherein the direct comparison uses an unsupervised machine-learning algorithm.

3. The method of claim 1, wherein the direct comparison uses a model-based algorithm.

4. The method of claim 1, further comprising:
segmenting the imaging dataset into voxels corresponding to a tissue of interest and voxels not of interest (104).

5. The method of claim 4, further comprising:
automatically analyzing the voxels of interest for the presence and extent of an imaging biomarker; and
comparing the presence and extent of the imaging biomarker in the voxels of interest to the presence and extent of the imaging biomarker in the historical imaging datasets.

6. The method of claim 5, wherein the imaging biomarker is selected from the group of parametric metrics consisting of: number of voxels among the voxels of interest with image intensity below or above a threshold intensity, percentage of voxels of interest with image intensity below or above a threshold intensity, mean image intensity of the voxels of interest, mean image intensity of the voxels among the voxels of interest with image intensity below or above a threshold intensity, standard deviation of the voxels of interest, standard deviation of the image intensity of the voxels among the voxels of interest with image intensity below or above a threshold intensity, other metrics derived from a histogram of the image voxel intensities for the voxels of interest, dimensions of an anatomical feature, pharamacokinetic modeling coefficients of the voxels of interest, and diffusion characteristics of the voxels of interest.

7. The method of claim 6, wherein the group of parametric metrics further includes the rate of change of any of the parametric metrics.

8. The method of claim 1, wherein the comparison comprises identifying similar imaging features between the patient's imaging dataset and the historical imaging datasets.

9. The method of claim 8, wherein the similar imaging features include one or any combination of: textural patterns of image intensity, statistical characteristics of the image intensity distribution, location of focal abnormalities, size of anatomical structure, size of abnormal structure, and physical characteristics of focal abnormalities.

10. The method of claim 1, wherein the calculation of personalized quantitative health status and risk metrics involves clinical data of the patient in addition to the medical image.

11. The method of claim 1, wherein image registration techniques are used to facilitate the comparison of the patient's imaging dataset to the historical imaging datasets.

12. The method of claim 1, wherein the origin of the historical datasets is selected from the group consisting of: a multi-center trial, archives of a facility where the patient's imaging dataset is acquired, archives of a facility where the patient is treated, and a purchasable database of imaging datasets and corresponding clinical outcomes.

13. The method of claim 1, wherein the comparison to historical datasets involves other types of clinical data of the patient in addition to the imaging dataset.

14. The method of claim 1, wherein the imaging dataset is from a modality selected from the group consisting of: magnetic resonance imaging, computed tomography, two-dimensional planar x-ray, x-ray mammography, positron emission tomography, ultrasound, and single-photon emission computed tomography.

15. The method of claim 1, further comprising:
automatically analyzing the imaging dataset for the presence and extent of an imaging biomarker; and
wherein directly comparing the imaging dataset comprises comparing the presence and extent of the imaging biomarker in the imaging dataset of the patient to the presence and extent of the imaging biomarker in historical imaging datasets (106) previously acquired from other patients having known clinical outcomes.

16. The method of claim 15, further comprising segmenting the imaging dataset of the patient into voxels corresponding to tissue of interest and voxels corresponding to tissue of no interest (104).

17. The method of claim 15, wherein the imaging biomarker is the number of voxels with intensity below a threshold.

18. The method of claim 17, wherein the threshold is in the range -910 HU to -960 HU.

19. The method of claim 15, wherein the quantitative health status and risk metrics are related to one or any combination of: myocardial infarction, Chronic Obstructive Pulmonary Disease COPD, emphysema, lung cancer, decreased lung function, COPD exacerbations, coronary artery disease, and stroke.

## Patentansprüche

1. Computerimplementiertes Verfahren zum Beurteilen und Kommunizieren des Zustands und Risikos der Gesundheit eines Patienten, wobei das Verfahren umfasst:
Empfangen eines Bildgebungsdatensatzes eines Patienten (102), wobei der Bildgebungsdatensatz eine Vielzahl von Voxeln umfasst;
direktes Vergleichen des Bildgebungsdatensatzes mit historischen Bildgebungsdatensätzen, die zuvor von anderen Patienten erfasst wurden, die bekannte klinische Ergebnisse (106) aufweisen;
Verwenden des Vergleichs, um personalisierte quantitative Metriken für Gesundheitszustand und Risiko für den Patienten (108) zu berechnen; und
Erstellen eines maßgeschneiderten Berichts auf Basis der Andeutungen von Charakteristiken des Benutzers, um die personalisierten quantitativen Metriken (110) für Gesundheit und Risiko zu kommunizieren.

2. Verfahren nach Anspruch 1, wobei der direkte Vergleich einen unüberwachten Algorithmus für Maschinenlernen verwendet.

3. Verfahren nach Anspruch 1, wobei der direkte Vergleich einen modellbasierten Algorithmus verwendet.

4. Verfahren nach Anspruch 1, ferner umfassend:
Segmentieren des Bildgebungsdatensatzes in Voxel, die einem Gewebe von Interesse und Voxel von keinem Interesse (104) entsprechen.

5. Verfahren nach Anspruch 4, ferner umfassend:
Automatisches Analysieren der Voxel von Interesse für die Anwesenheit und das Ausmaß eines bildgebenden Biomarkers; und
Vergleichen der Anwesenheit und des Ausmaßes des Biomerkers in den Voxeln von Interesse mit der Anwesenheit und dem Ausmaß des bildgebenden Biomarkers in den historischen Bildgebungsdatensätzen.

6. Verfahren nach Anspruch 5, wobei der bildgebende Biomarker aus der Gruppe von parametrischen Metriken selektiert wird, bestehend aus: Zahl von Voxeln von Interesse mit Bildintensität unterhalb oder oberhalb einer Schwellenwertintensität, Prozentsatz von Voxeln von Interesse mit Bildintensität unterhalb oder oberhalb einer Schwellenwertintensität, mittlerer Bildintensität der Voxel von Interesse, mittlere Bildintensität der Voxel unter den Voxeln von Interesse mit Bildintensität unterhalb oder oberhalb einer Schwellenwertintensität, Standardabweichung der Voxel von Interesse, Standardabweichung der Bildintensität der Voxel unter den Voxeln von Interesse mit Bildintensität unterhalb oder oberhalb einer Schwellenwertintensität, anderen Metriken, die aus einem Histogramm der Intensitäten von Bildvoxeln für die Voxel von Interesse abgeleitet sind, Abmessungen eines anatomischen Merkmals, pharamakokinetischen Modellierungskoeffizienten der Voxel von Interesse und Diffusionskennlinien der Voxel von Interesse.

7. Verfahren nach Anspruch 6, wobei die Gruppe von parametrischen Metriken eine Änderungsrate irgendeiner der parametrischen Metriken einschließt.

8. Verfahren nach Anspruch 1, wobei der Vergleich das Identifizieren ähnlicher bildgebender Merkmale zwischen dem Bildgebungssatz und den historischen Bildgebungsdatensätzen des Patienten umfasst.

9. Verfahren nach Anspruch 8, wobei die ähnlichen Bildgebungsmerkmale eine oder irgendeine Kombination einschließt von: strukturellen Mustern von Bildintensität, statistischen Kennlinien der Verteilung der Bildintensität, Position von herdförmigen Abnormalitäten, Größe der anatomischen Struktur, und physikalische Kennlinien von herdförmigen Abnormalitäten.

10. Verfahren nach Anspruch 1, wobei die Berechnung des personalisierten quantitativen Gesundheitszustands und der Risikometriken klinische Daten des Patienten zusätzlich zum medizinischen Bild involviert.

11. Verfahren nach Anspruch 1, wobei Bildregistrierungstechniken verwendet werden, um den Vergleich des Bildgebungsdatensatzes des Patienten mit den historischen Bildgebungsdatensätzen zu erleichtern.

12. Verfahren nach Anspruch 1, wobei die Quelle der historischen Datensätze aus der Gruppe selektiert wird, bestehen aus: Einer Multi-Center-Studie, Archiven einer Einrichtung wo der Bildgebungsdatensatz des Patienten erfasst wird, Archiven einer Einrichtung wo der Patient behandelt wird und einer käuflichen Datenbank von Bildgebungsdatensätzen und entsprechenden klinischen Ergebnissen.

13. Verfahren nach Anspruch 1, wobei der Vergleich mit historischen Datensätzen andere Arten klinischer Daten des Patienten zusätzlich zum Bildgebungsdatensatz involviert.

14. Verfahren nach Anspruch 1, wobei der Bildgebungsdatensatz von einer Modalität abstammt, die aus der Gruppe selektiert ist, bestehend aus: Kernspintomografie, Computer-Tomografie, zweidimensionales planares Röntgen, Röntgen-Mammografie, Positronen-Emissionstomografie, Ultraschall und Einzelphotonen-Emissionscomputertomografie.

15. Verfahren nach Anspruch 1, ferner umfassend:
Automatisches Analysieren des Bildgebungsdatensatzes für die Anwesenheit und das Ausmaß eines bildgebenden Biomarkers; und
wobei direktes Vergleichen des Bildgebungsdatensatzes das Vergleichen der Anwesenheit und des Ausmaßes des bildgebenden Biomarkers im Bilddatensatz des Patienten mit der Anwesenheit und dem Ausmaß des bildgebenden Biomarkers in historischen Bildgebungsdatensätzen (106) umfasst, die zuvor von anderen Patienten mit bekannten klinischen Ergebnissen erfasst wurden.

16. Verfahren nach Anspruch 15, das ferner das Segmentieren des Bildgebungsdatensatzes des Patienten in Voxel, die Gewebe on Interesse entsprechen und Voxel umfasst, die Gewebe von keinem Interesse (104) entsprechen.

17. Verfahren nach Anspruch 15, wobei der bildgebende Biomarker die Zahl von Voxel mit Intensität unter einen Schwellenwert ist.

18. Verfahren nach Anspruch 17, wobei der Schwellenwert im Bereich -910 HU bis -960 HU liegt.

19. Verfahren nach Anspruch 15, wobei sich der quantitative Gesundheitszustand und die Risikometriken auf eine oder irgendeine Kombination von: myokardialem Infarkt, chronisch obstruktive Lungenerkrankung, COPD, Emphysem, Lungenkrebs, verringerte Lungenfunktion, COPD-Verschlimmerungen, koronare Arterienkrankheit und Schlaganfall.

## Revendications

1. Procédé mis en oeuvre par ordinateur pour évaluer et communiquer l'état de santé et le risque associé à la santé d'un patient, le procédé consistant à :
recevoir un ensemble de données d'imagerie d'un patient (102), l'ensemble de données d'imagerie comportant une pluralité de voxels ;
comparer directement l'ensemble de données d'imagerie à un ensemble de données d'imagerie historiques précédemment obtenus d'autres patients ayant des résultats cliniques connus (106) ;
utiliser la comparaison pour calculer des paramètres d'état et de risque de santé quantitatifs personnalisés du patient (108) ; et
établir un rapport sur mesure basé sur des indications des caractéristiques de l'utilisateur, pour communiquer les paramètres d'état et de risque de santé quantitatifs personnalisés du patient (110).

2. Procédé selon la revendication 1, dans lequel la comparaison directe fait appel à un algorithme d'apprentissage machine non supervisé.

3. Procédé selon la revendication 1, dans lequel la comparaison directe fait appel à un algorithme basé sur un modèle.

4. Procédé selon la revendication 1, consistant en outre à :
segmenter l'ensemble de données d'imagerie en voxels présentant un grand intérêt et en voxels sans intérêt (104).

5. Procédé selon la revendication 4, consistant en outre à :
analyser automatiquement les voxels d'intérêt pour la présence et l'étendue d'un biomarqueur d'imagerie ; et
comparer la présence et l'étendue du biomarqueur d'imagerie chez les voxels d'intérêt à la présence et l'étendue du biomarqueur d'imagerie dans les ensembles de données d'imagerie historiques.

6. Procédé selon la revendication 5, dans lequel le biomarqueur d'imagerie est sélectionné dans le groupe de paramètres consistant en : nombre de voxels parmi les voxels d'intérêt ayant une intensité d'image inférieure ou supérieure à une intensité de seuil, pourcentage de voxels d'intérêt ayant une intensité d'image inférieure ou supérieure à une intensité de seuil, une intensité d'image moyenne des voxels d'intérêt, une intensité d'image moyenne des voxels parmi les voxels d'intérêt ayant une intensité d'image inférieure ou supérieure à une intensité de seuil, un écart type des voxels d'intérêt, un écart type de l'intensité d'image des voxels parmi les voxels d'intérêt ayant une intensité d'image inférieure ou supérieure à une intensité de seuil, d'autres paramètres déterminés à partir d'un histogramme des intensités des voxels d'image pour les voxels d'intérêt, des dimensions d'une caractéristique anatomique, des coefficients de modélisation pharamacocinétique des voxels d'intérêt, et des caractéristiques de diffusion des voxels d'intérêt.

7. Procédé selon la revendication 6, dans lequel le groupe de paramètres inclut en outre le taux de changement des paramètres.

8. Procédé selon la revendication 1, dans lequel la comparaison consiste à identifier des caractéristiques d'imagerie similaires entre l'ensemble de données d'imagerie du patient et l'ensemble de données d'imagerie historiques.

9. Procédé selon la revendication 8, dans lequel les caractéristiques d'imagerie similaires incluent une combinaison ou une quelconque combinaison de : schémas texturaux d'intensité d'image, caractéristiques statistiques de la distribution de l'intensité d'image, localisation des anomalies focales, dimension de la structure anatomique, dimension de la structure anormale, et caractéristique physique des anomalies focales.

10. Procédé selon la revendication 1, dans lequel le calcul des paramètres d'état et de risque de santé quantitatifs personnalisés implique des données cliniques du patient en plus de l'image médicale.

11. Procédé selon la revendication 1, dans lequel les techniques d'enregistrement d'images sont utilisées pour faciliter la comparaison des ensembles de données d'imagerie du patient aux ensembles de données d'imagerie historiques.

12. Procédé selon la revendication 1, dans lequel l'origine des ensembles de données historiques est sélectionnée dans le groupe consistant en : un essai multicentrique, des archives d'un endroit où les ensembles de données d'imagerie sont obtenus, des archives d'un endroit où est traité le patient, et une base de données achetable d'ensembles de données d'imagerie et de résultats cliniques correspondants.

13. Procédé selon la revendication 1, dans lequel la comparaison avec des ensembles de données historiques implique d'autres types de données cliniques du patient en plus de l'ensemble de données d'imagerie.

14. Procédé selon la revendication 1, dans lequel l'ensemble de données d'imagerie provient d'une modalité sélectionnée dans le groupe consistant en : imagerie par résonance magnétique, tomographie informatisée, rayon X planaire bidimensionnel, mammographie par rayons X, tomographie par émission de positrons, ultrasons et tomographie informatisée par émission monophotonique.

15. Procédé selon la revendication 1, consistant en outre à :
analyser automatiquement l'ensemble de données d'imagerie pour la présence et l'étendue d'un biomarqueur d'imagerie ; et
dans lequel la comparaison directe de l'ensemble de données d'imagerie comprend la comparaison de la présence et de l'étendue du biomarqueur d'imagerie dans l'ensemble de données d'imagerie du patient avec la présence et l'étendue du biomarqueur d'imagerie dans des ensembles de données d'imagerie historiques (106) précédemment obtenus d'autres patients ayant des résultats cliniques connus.

16. Procédé selon la revendication 15, consistant en outre à segmenter l'ensemble de données d'imagerie du patient en voxels présentant un grand intérêt et en voxels sans intérêt (104) .

17. Procédé selon la revendication 15, dans lequel le biomarqueur est le nombre de voxels ayant une intensité inférieure à un seuil.

18. Procédé selon la revendication 17, dans lequel le seuil est compris dans la plage de -910 HU et -960 HU.

19. Procédé selon la revendication 15, dans lequel les paramètres d'état et de risque de santé quantitatifs sont liés à une combinaison ou à une quelconque combinaison : d'un infarctus du myocarde, d'une maladie pulmonaire obstructive chronique MPOC, d'un emphysème, d'un cancer du poumon, d'un affaiblissement de la fonction pulmonaire, d'une MPOC exacerbée, d'une maladie coronarienne et d'une attaque cérébrale.
